# EUROPÄISCHE PATENTSCHRIFT

(11) **EP 1 514 645 B1**
(45) Veröffentlichungstag und Bekanntmachung des Hinweises auf die Patenterteilung: **28.06.2006**
(21) Anmeldenummer: 04011866.3
(22) Anmeldetag: 19.05.2004
(51) Int. Cl.: B25B 23/142, B25B 15/02, A61B 17/88

(54) **Schraubwerkzeug mit einer Vorrichtung zur Begrenzung des übertragenen Drehmomentes**
Screwdriver with torque limiting clutch
Tournevis avec embrayage limitateur de couple

(30) Priorität: 10.09.2003 DE 10341697
(43) Veröffentlichungstag der Anmeldung: 16.03.2005
(73) Patentinhaber: FELO-Werkzeugfabrik Holland-Letz GmbH, 35279 Neustadt (DE)
(72) Erfinder: Bubel, Rainer, 35279 Neustadt/Hessen (DE)
(74) Vertreter: Buchhold, Jürgen

(56) Entgegenhaltungen:
- EP-A- 1 092 510
- DE-A- 2 427 352
- DE-A- 2 647 996
- DE-C- 884 780
- US-A- 2 335 574

## Beschreibung

Die Erfindung betrifft ein Schraubwerkzeug mit einer Vorrichtung zum Einstellen eines vorgewählten Drehmomentes, bei dessen Erreichen die Übertragung des Drehmomentes von dem Antriebsteil in das Abtriebsteil gemäß dem Oberbegriff des Anspruchs 1 unterbrochen wird. Vorrichtungen dieser Gattung sind in relativ vielen Ausführungsarten bekannt, sowohl zum Einsatz bei Kraftschraubern als auch bei Hand- Schraubwerkzeugen. Bei den bekannten Vorrichtungen kommen verschiedene Systeme zur Anwendung.

Von besonderem Interesse sind im Bereich der Erfindung solche Systeme, bei denen das Antriebsteil achszentrisch zum Abtriebsteil angeordnet ist.

Die im Vergleich mit dem Erfindungsgegenstand zu beachtenden Systeme werden beispielhaft mit ihrem Funktionsprinzip beschrieben:

Ein Schraubwerkzeug entsprechend dem Oberbegriff des Anspruchs 1 ist beispielsweise aus der US 5,746,298 bekannt, wobei dieser einen komplizierten Aufbau der Kupplungsmechanik aufweist, deren Teile aus Metall bestehen. In einem Griff ist ein Schaft gelagert, der Schaft weist ein sechseckiges Endstück auf, das von fingerartigen Segmenten einer in Längsrichtung teilweise geschlitzten Hülse umgriffen wird. Ein in Längsrichtung in der Höhlung des Griffes verstellbarer Kragen umschließt die fingerartigen Segmente und wirkt mit einer Radialkraft auf diese Weise ein, eine entsprechende Radialkraft wirkt von den Enden der fingerartigen Segmente auf das sechseckige Endstück des Schaftes. Je näher der Kragen am Ende der fingerartigen Segmente steht, um so geringer ist deren Möglichkeit, radial auszufedern und ein Durchdrehen des sechseckigen Endstückes zu erlauben - um so höher ist also das Auslöse-Drehmoment. Die Kupplung ist teuer in der Herstellung. Zum Voreinstellen eines Auslösedrehmomentes muss der Griff teilweise demontiert werden, (Spalte 6, Zeilen 42 bis 49), das System ist zu umständlich für Einsatz mit wechselnden Drehmomenten.

Aus OS 26 47 996 ist ein System bekannt, das im wesentlichen aus Axial-Kugel- oder Rollenlagern besteht, welche durch eine Schraubenfeder vorgespannt sind. Die Kugeln oder Rollen sitzen dabei in in den Lagerscheiben eingeformten Rastmulden und werden dort durch die Vorspannung gehalten. Eine der Lagerscheiben ist mit dem Antriebsteil verbunden, die andere mit dem Abtriebsteil. Übersteigt das Drehmoment einen vorgegebenen Wert, so wird die in Umfangsrichtung wirkende Kraft größer als die durch die Vorspannung der Feder im Sitz der Kugel oder Rollen wirkende form- und reibungsschlüssige Kraft, die Kugeln oder Rollen steigen aus den Rastmulden, die Lagerscheiben verdrehen sich gegeneinander, die DrehmomentÜbertragung wird unterbrochen.

Bei einer Vorrichtung gemäß OS 24 27 352 besteht das Antriebsteil aus einem Schaft mit einer tellerartigen Vergrößerung des Schaftes an einem Ende. Die tellerartige Vergrößerung weist an der Stirnseite in Umfangsrichtung eine wellenförmige Verzahnung auf und bildet ein Mitnehmer-Element. Das zweite Mitnehmer-Element bildet eine gleiche tellerartige Vergrößerung mit Verzahnung am Schaftende des Abtriebsteiles. Durch eine Druckfeder, ein Paket von Tellerfedern oder eine Schraubenfeder, werden die Mitnehmer - Elemente aufeinander gepreßt.

Aus OS 101 43 181 A1 ist eine Vorrichtung zur Begrenzung des von einem Schraubwerkzeug übertragbaren Drehmomentes bekannt. Die Kupplung zwischen Antriebsteil - dem Griff- und Abtriebsteil - dem Schaft - besteht im wesentlichen aus einer Käfighülse mit schlitzartigen Durchbrechungen der Hülsenwand und Kugeln, die mit einem Teil ihres Körpers in die Schlitze hineinragen, mit dem Hauptteil des Körper jedoch in Rastvertiefungen von Andrucktellern eingreifen, welche ihrerseits durch eine Druckfeder über die Kugeln gegeneinander verspannt sind. Bei Überschreiten eines durch die Vorspannung der Feder vorgegebenen Drehmomentes werden die Kugeln aus den Rastvertiefungen in die Durchbrechungen der Hülsenwand gedrückt, die Drehmomentübertragung wird unterbrochen. Die Konstruktion entspricht im Grundprinzip der in OS 26 47 996 beschriebenen Konstruktion.

Die Konstruktion gemäß OS 101 43 181 A1 ist in der Herstellung teuer, weil die Kupplung aus Präzisionsteilen aus Metall besteht.

Bei einem Schraubendreher mit Drehmomentbegrenzung gemäß EP 1 092 510 A2 weist ein Schaft eine ballige Endfläche auf zur Übertragung einer Axialkraft auf die innere Oberfläche des oberen Griffteiles, die Axialkraft einer Schraubenfeder wird in Richtung zum oberen Griffteil auf eine Buchse übertragen welche drehbar auf dem Schaft aufgesteckt ist. Die Buchse weist auf der zum oberen Griffteil hingewendeten Stirnseite eine Verzahnung und am Umfang Nuten auf, in die Rippen eingreifen, welche von der Innenseite der Griffhöhlung radial vorstehen. Über diese Rippen wird ein Drehmoment vom Griff in die Buchse eingeleitet und über die stirnseitige Verzahnung in eine zweite Verzahnung, die auf der Unterseite einer tellerartigen Vergrößerung des Schaftes eingeformt ist. Die beiden Verzahnungen weisen in einer Richtung abgeschrägte, in der anderen Richtung gerade, parallel zur Achse des Schaftes verlaufende Flanken auf.
Wird ein Drehmoment in Richtung der schräg verlaufenden Zahnflanken aufgebracht, so hebt sich die Verzahnung der Buchse aus der Verzahnung am Teller des Schaftes, wenn ein durch die Federspannung eingestelltes Drehmoment überschritten wird. Dadurch wird die Einleitung des Drehmomentes vom Griff in den Schaft unterbrochen. Nachteilig ein dieser Konstruktion ist, dass die Axialkraft der Feder über die Verzahnung auf den Teller des Schaftes und somit über seine ballige Endfläche auf die innere Oberfläche des Griffes wirkt. Diese Axialkraft überlagert sich mit der Axialkraft, die bei Benutzung des Schraubendrehers von der Hand über den Griff und den Schaft in die Klinge des Schraubendrehers eingeleitet wird. Das hat zur Folge, dass die Reibung im Bereich der Anlagefläche der Endfläche des Schaftes mit der inneren Oberfläche des Griffes relativ hoch ist. Infolgedessen wird das Auslöse-Drehmoment nicht alleine durch die Federspannung, sondern auch durch die von der Hand ausgeübten Axialkraft beeinflußt, das gewünschte Auslösedrehmoment wird in der Praxis nicht mit Genauigkeit eingehalten.

Vorteilhaft ist zwar, dass die wesentlichen Teile aus Kunststoff im Spritzgießverfahren hergestellt sind, nachteilig ist jedoch, dass bei dieser Konstruktion nur relativ niedrige Drehmomente übertragen werden können. Bei höheren Drehmomenten treten plastische Verformungen an wichtigen Elementen der Mechanik auf, die Auslösegenauigkeit vermindert sich stark, unter Umständen ist die Funktion überhaupt nicht mehr gewährleistet.

Die bekannten Ausführungen sind relativ teuer in der Herstellung, weil sie im Aufbau kompliziert und die Teile zumeist aus Metall hergestellt sind. Auch ist die Einstellung oft nicht einfach durchzuführen und die Ungenauigkeit beim Auslösen zu hoch. In neuerer Zeit erhöht sich der Bedarf für Schraubwerkzeuge mit einstellbaren Höchstwerten für das übertragene Drehmoment, wobei der Auslöse-Drehmoment in einem gewissen Bereich variabel einstellbar sein soll. Anwendungsgebiete sind die Montage, auch im Service, von elektrischen Bauteilen, die Verschraubung von Kunststoffteilen und die Befestigung von Schneidplatten von Zerspanungswerkzeugen. Mit dem steigenden Bedarf ist die Forderung der Anwender verbunden, derartige Schraubwerkzeuge kostengünstiger anzubieten.

Die Aufgabe ist es, ein Schraubwerkzeug mit einer Vorrichtung zur Begrenzung eines vorgewählten Drehmomentes zu entwickeln, das kostengünstig herstellbar ist, mit Drehmomenten der im Anwendungsbereich geforderten Höhe belastet werden kann, schnell verstellbar ist und dessen Funktionsgenauigkeit über eine lange Gebrauchsdauer erhalten bleibt. Diese Aufgabe wird durch den Erfindungsgegenstand mit den Merkmalen von Anspruch 1 gelöst.
Die wesentlichen Merkmale des Erfindungsgegenstandes sind: die von einem Griff als Antriebsteil in die Klinge als Abtriebsteil einzuleitenden Drehmomente werden an zwei Stellen in eine Drehmoment-Begrenzungsmechanik übertragen, die zwei auf einer Zahnhülse angeordneten Kupplungen aufweist, welche durch eine zwischen den Kupplungen angeordnete Druckfeder, vorzugsweise eine Schraubenfeder, mit einer Vorspannung beaufschlagt werden. Die Kupplungen bestehen aus zwei zylindrischen Teilen.

Je ein Teil der beiden Kupplungen, die Rastbuchse, ist durch eine Innenverzahnung mit der Außenverzahnung der Zahnhülse formschlüssig drehfest und verschiebbar verbunden. Das andere Teil der Kupplungen, die Mitnehmerbüchse, ist durch eine Verzahnung drehfest mit dem Griff verbunden, die Zahnhülse ist über die Zahnkamm- Flächen in einer Bohrung der Mitnehmerbüchse drehbar geführt. Die einander zugekehrten Stirnseiten der Kupplungsteile weisen ineinandergreifende Verzahnungen auf. Durch die Vorspannung der Feder werden die Verzahnungen in Eingriff gehalten, bis das über die mit dem Griff verbundenen Mitnehmerbüchse eingeleitete Drehmoment so groß ist, dass es den in der Verzahnung wirkenden Reibungs-Formschluß löst, die Zähne sich gegeneinander verdrehen und außer Eingriff kommen, so daß die Übertragung des Drehmomentes unterbrochen wird. Die ineinander greifende Verzahnung von Rastbuchsen und Mitnehmerbüchsen wird zweckmäßigerweise in ihrem Zahnprofil so gewählt, dass die Zähne in Drehrichtung beim Einschrauben unter einem Winkel α schräg ansteigende Zahnflanken aufweisen, die Zahnkanten leicht abgerundet und die Zahnkämme abgeflacht oder ebenfalls abgerundet sind. Die Zahnflanken in Drehrichtung zum Lösen von Schrauben verlaufen dagegen unter einem Winkel von 0° oder unter einem sehr kleinen Winkel, bezogen auf die Drehachse der Vorrichtung, da beim Ausdrehen von Schrauben das erforderliche Drehmoment nur durch den mehr oder weniger festen Sitz der Schraube bestimmt wird, nicht aber durch eine definierte Vorgabe. Außerdem wird bei einem Flankenwinkel von 0° vermieden, dass die Schraube beim Wiedereingreifen der Verzahnungen über schräge Flanken wieder zurückgedreht wird.
Zum Einstellen des Auslöse- Drehmomentes wird die hintere Kupplung axial verschoben, indem auf eine in Umfangsrichtung schräg ansteigende Ringfläche an der hinteren Stirnseite der Mitnehmerbüchse die in gleicher Weise ansteigende Stirnseite einer Verstellscheibe einwirkt, die von dem Hinterende des Griffes aus verdreht werden kann. Durch die axiale Verschiebung der hinteren Kupplung verändert sich die Vorspannung der Druckfeder, dadurch der Reibungs- Formschluß in der Verzahnung der Kupplungen und somit die Höhe des Auslöse- Drehmomentes. Die Rückseite der Verstellscheibe ist in Umfangsrichtung mit einer relativ feinen Stirnverzahnung versehen. Am Boden der Höhlung der Kappe ist eine Rastscheibe eingesetzt und über eine Umfangsverzahnung drehfest mit der Kappe verbunden. Auf der Verstellscheibe zugewendeten Seite weist die Rastscheibe eine Stirnverzahnung auf, die der Stirnverzahnung an der Rückseite der Verstellscheibe entspricht. Die Axialkraft der Feder presst über hintere Kupplung die Verstellscheibe mit ihrer Verzahnung in die Verzahnung der Rastscheibe. Im Zusammenwirken der beiden Verzahnungen werden die jeweiligen Positionen fixiert, die sich beim Einstellen eines gewünschten Auslösedrehmomentes durch Verdrehen der Verstellscheibe ergeben.
In die Zahnhülse ist die Klinge eines Schraubendrehers oder ein Schaft mit Aufnahmekopf für auswechselbare Werkzeuge fest oder auswechselbar eingesetzt. Bei Drehung der Zahnhülse werden die Klinge oder der Schaft von der Zahnhülse mitgenommen.
Die Zahnhülse stützt sich axial am Grund einer Bohrung in der Verstellscheibe ab. Die beim Gebrauch des Schraubwerkzeuges von der Hand des Benutzers ausgeübte Axialkraft wird somit von Griff / Kappe über die Rastscheibe und die Verstellscheibe auf die Zahnhülse übertragen. Die Vorspannkraft der Feder wirkt nur auf die beiden Kupplungen, nicht jedoch auch auf das Axiallager der Zahnhülse.

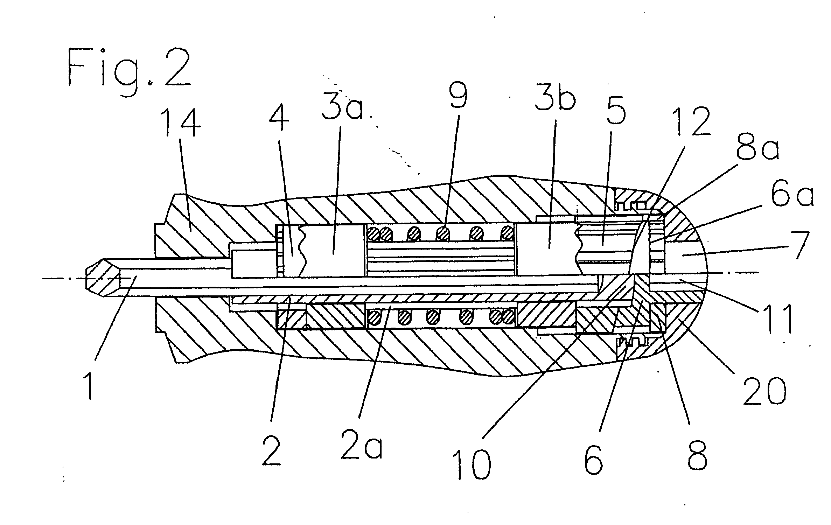

Der Erfindungsgegenstand wird durch die Zeichnungen beispielhaft dargestellt.
Es zeigen:

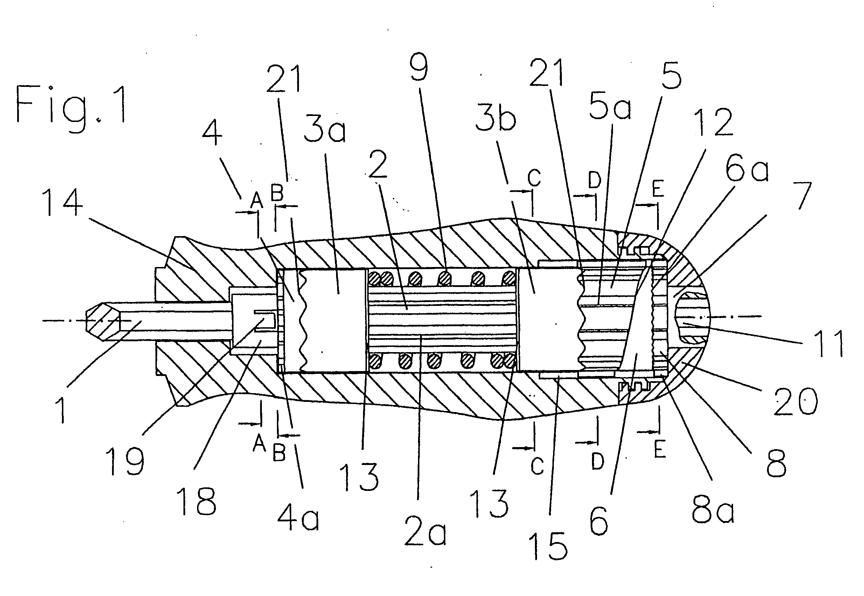

- Fig. 1: einen Längsschnitt durch das Schraubwerkzeug mit einer Vorrichtung zur Begrenzung eines vorgewählten Drehmomentes mit den offenliegenden Teilen der Vorrichtung.
Darin sind:
(1) der Schaft (Abtriebsteil)
(2) die Zahnhülse mit Außenverzahnung
(2a) die Außenverzahnung der Zahnhülse
(3a) die erste Rastbuchse, der ersten Kupplung
(3b) die zweite Rastbuchse, der zweiten Kupplung
(4) die erste Mitnehmerbüchse, der ersten Kupplung
(4a) die Stirnverzahnung der ersten Mitnehmerbüchse
(5) die zweite Mitnehmerbüchse der zweiten Kupplung
(5a) die Außenverzahnung der zweiten Mitnehmerbüchse (5)
(21) die Stirnverzahnung an den Rastbuchsen (3a, 3b) und Mitnehmerbüchsen (4, 5)
(6) die Verstellscheibe
(6a) eine feine Stirnverzahnung an den einander zugewandten Stirnseiten der Verstellscheibe (6) und der Rastscheibe (8)
(7) ein mit der Verstellscheibe einstückig verbundener Zapfen
(8) die Rastscheibe
(8a) die Umfangsverzahnung der Rastscheibe (8)
(9) die Druckfeder (Schraubenfeder)
(11) eine in den Zapfen (7) eingeformte Höhlung
(12) in Umfangsrichtung schräg ansteigende Ringflächen
(13) Stahlscheiben
(14) der Griff
(15) die Innenverzahnung im Griff
(18) eine Klemmeinrichtung zum axialen Halten eines in der Zahnhülse (2) auswechselbar gelagerten Schaftes (1)
(19) eine Federzunge oder Federsattel in der Klemmeinrichtung, einstückig eingeformt
(20) die Kappe
- Fig. 2: einen zweiten Längsschnitt, der in der unteren Hälfte bis zu dem in der Zahnhülse gelagerten Schaft führt.
Darin sind:
(1) der in der Zahnhülse (2) gelagerte Schaft
(2) die Zahnhülse mit Außenverzahnung
(2a) die Außenverzahnung der Zahnhülse
(3a) die erste Rastbuchse, der ersten Kupplung
(3b) die zweite Rastbuchse, der zweiten Kupplung
(4) die erste Mitnehmerbüchse, der ersten Kupplung
(4a) eine Stirnverzahnung der ersten Mitnehmerbüchse
(5) die zweite Mitnehmerbüchse
(5a) die Außenverzahnung der zweiten Mitnehmerbüchse (5)
(21) die Stirnverzahnung an den Rastbuchsen (3a, 3b) und Mitnehmerbüchsen (4, 5)
(6) die Verstellscheibe
(6a) eine feine Stirnverzahnung an den einander zugewandten Stirnseiten der Verstellscheibe (6) und der Rastscheibe (8)
(7) ein mit der Verstellscheibe einstückig verbundener Zapfen
(8) die Rastscheibe
(8a) die Umfangsverzahnung der Rastscheibe (8)
(9) die Druckfeder (Schraubenfeder)
(10) der Endabschnitt der Zahnhülse (2)
(11) eine in den Zapfen (7) eingeformte Höhlung
(12) in Umfangsrichtung schräg ansteigende Ringflächen
(14) der Griff
(20) die Kappe
- Fig. 3: einen Querschnitt entlang der Linie A-A in Fig. 1 durch den Bereich der Klemmvorrichtung zum axialen Halten eines in der Höhlung der Zahnhülse auswechselbar gelagerten Schaftes, die linke Seite stellt die Situation mit eingesetztem Schaft dar, die rechte Seite ohne eingesetzten Schaft
Darin sind:
(1) der Schaft (Abtriebsteil)
(2b) die Höhlung der Zahnhülse (2)
(14) der Griff
(18) die Klemmeinrichtung
(19) die Federzunge der Klemmeinrichtung
- Fig. 4.: einen Querschnitt entlang der Linie B-B in Fig. 1 ohne in die Zahnhülse (2) eingesetzten Schaft (1).
Darin sind:
(2) die Zahnhülse mit Außenverzahnung
(2a) die Außenverzahnung der Zahnhülse
(2b) die Höhlung der Zahnhülse (2)
(4a) die Stirnverzahnung der ersten Mitnehmerbüchse (4)
(14) der Griff
- Fig. 5.: einen Querschnitt entlang der Linie C-C in Fig. 1 ohne in die Zahnhülse (2) eingesetzten Schaft (1).
Darin sind:
(2) die Zahnhülse mit Außenverzahnung
(2a) die Außenverzahnung der Zahnhülse
(2b) die Höhlung der Zahnhülse (2)
(3b) die zweite Rastbuchse
(3d) die Innenverzahnung der zweiten Rastbuchse (3b)
(14) der Griff
- Fig. 6.: einen Querschnitt entlang der Linie D-D in Fig. 1 ohne in die Zahnhülse (2) eingesetzten Schaft (1).
Darin sind:
(2) die Zahnhülse mit Außenverzahnung
(2a) die Außenverzahnung der Zahnhülse
(2b) die Höhlung der Zahnhülse (2)
(5) die zweite Mitnehmerbüchse
(5a) die Umfangsverzahnung der zweiten Mitnehmerbüchse
(14) der Griff
(15) die Innenverzahnung des Griffes
- Fig. 7.: einen Querschnitt entlang der Linie E-E in Fig. 1.
Darin sind:
(7) der Zapfen der Stellscheibe (6)
(8) eine Rastscheibe
(8a) die Umfangsverzahnung der Rastscheibe (8)
(14) der Griff
(20) die Kappe
- Fig. 8.: eine Ansicht von hinten auf die Kappe des Griffes mit der Skalierung für die Auslöse-Drehmoment-Einstellung
Darin sind:
(7) der Zapfen
(11) das Innenprofil im Zapfen
(14) der Griff
(16) die Markierung auf dem Zapfen (7)
(17) die Skala auf der Kappe (20)
(20) die Kappe
- Fig. 9: eine Seitenansicht der Rastbuchsen 3a und 3b sowie der Mitnehmerbuchsen 4 und 5.
Darin ist:
(21a) eine Verzahnung mit ungleich schrägen Zahnflanken
(α) der Flankenwinkel bezogen auf die Drehachse
- Fig. 10: eine Seitenansicht der Rastbuchsen 3a und 3b sowie der Mitnehmerbüchsen 4 und 5.
Darin ist:
(21b) eine Verzahnung, bei der der Winkel der Zahnflanken in einer Drehrichtung 0° in Bezug auf die Drehachse beträgt
- Fig. 11: zeigt einen Längsschnitt in dem die hintere Kupplung weiter nach vorne verschoben und die Feder stärker vorgespannt ist.
Darin sind:
(3a, 3b) die erste und zweite Rastbuchse
(4) die zweite Mitnehmerbüchse
(6) eine Verstellscheibe
(7) ein mit der Verstellscheibe einstückig verbundener Zapfen
(9) eine Druckfeder (Schraubenfeder)
(11) eine in den Zapfen (7) eingeformte Höhlung
- Fig. 12: zeigt einen vergrößerten Ausschnitt der wellenförmigen Verzahnung (21) an den Rastbuchsen (3a, 3b) und Mitnehmerbüchsen (4, 5)

Wie Fig. 1 und 2 zeigen, besteht der Drehmoment-Schraubendreher gemäß der Erfindung aus einer Klinge, deren Schaft (1) einen unrunden Profilquerschnitt - zum Beispiel einen Sechskant-Profilquerschnitt - aufweist, in einer Zahnhülse (2) sitzt und bis kurz vor deren hinteres Ende reicht. Die Zahnhülse (2) weist am Umfang eine im wesentlichen über die Länge laufende Verzahnung (2a) auf, wobei die Zähne vorzugsweise ein Rechteck-Zahnprofil haben. Auf die Zahnhülse (2) sind die vordere erste und hintere zweite Rastbuchse (3a, 3b) der ersten und zweiten Kupplung aufgesteckt. Die beiden Rastbuchsen (3a, 3b) dienen mit ihrer zylindrischen Umfangsfläche auch als Führung und zur radialen Abstützung der nachfolgend beschriebenen Mechanik in der Höhlung des Griffes (14), welche durch die Kappe (20) verschlossen ist.
Mit einer Innenverzahnung (3d Fig. 5) greifen die Rastbuchsen in die Verzahnung (2a) der Zahnhülse (2) drehfest ein. An einer Stirnseite weisen die Rastbuchsen eine Verzahnung (21) auf, die ein wellenförmiges oder an den Spitzen abgerundetes Zahnprofil hat. Die Verzahnung (21) greift in eine entsprechende Verzahnung der Mitnehmerbüchsen (4, 5) der ersten und zweiten Kupplung ein. Die erste Mitnehmerbüchse (4) weist an der der Verzahnung (21) gegenüberliegenden Stirnseite eine Rechteck-Stirnverzahnung (4a) auf, die in eine gleichförmige Verzahnung im Griff (14) spielfrei eingreift. Dies ist die eine Stelle, an der das Drehmoment vom Griff in die Drehmoment-Begrenzungs-Mechanik eingeleitet wird. Zwischen den Rastbuchse (3a, 3b) ist eine Druckfeder (9) vorgespannt angeordnet, wobei zwei Stahlscheiben (13) die Auflage für die Schraubenfeder bilden. Die zweite Mitnehmerbüchse (5) weist an der der Verzahnung (21) gegenüberliegenden Stirnseite eine in Umfangsrichtung ansteigende Ringfläche (12) auf. Diese Ringfläche liegt an einer gleichförmigen Ringfläche einer Verstellscheibe (6) an. Am Umfang weist die zweite Mitnehmerbüchse (5) eine Verzahnung (5a) auf, mit der sie drehfest und verschiebbar in eine entsprechende Innenverzahnung (15) in der Höhlung des Griffes (14) eingreift. Die ist die zweite Stelle, an der das Drehmoment vom Griff in die Drehmomen-Begrenzung-Mechanik eingeleitet wird. Die Verstellscheibe (6) ist an der der schräg aufsteigenden Ringfläche (12) gegenüberliegenden Stirnseite mit einer feinen Verzahnung (6a) versehen und weist auf dieser Seite weiterhin einen Zapfen (7) auf, der in Bohrungen in der Rastscheibe (8) und der Kappe (20) des Griffes geführt ist. In den Zapfen (7) ist im Beispiel ein Innensechskantprofil (11) eingeformt, in das ein Schlüssel zum Verstellen der Verstellscheibe (6) eingreifen kann. Zwischen der Verstellscheibe (6) und der Kappe (20) ist eine Rastscheibe (8) angeordnet, die am Außenumfang eine Rechteck-Verzahnung (8a) aufweist, welche in eine gleichförmige Innenverzahnung im Griff (14) eingreift. Die der Verstellscheibe (6) zugewandte Stirnseite der Rastscheibe (8) weist ebenfalls eine feine Verzahnung (6a) auf, die in die gleichförmige Verzahnung der Verstellscheibe (6) eingreift.
Durch Drehen der Verstellscheibe (6) wird über die ansteigende Ringfläche (12) die zweite Mitnehmerbüchse (5) zusammen mit der zweiten Rastbuchse (3b) axial verschoben und dadurch die Vorspannung der Schraubenfeder (9) verändert. Wird über den Griff (14) und dessen Innenverzahnungen (4a, 15) ein Drehmoment auf die Mitnehmerbüchsen (4, 5) übertragen, so wird dieses Drehmoment über die Rastbuchsen (3a, 3b) auf die Zahnhülse (2) und von dieser auf den Schaft (1) weitergeleitet. Die Vorspannung der Schraubenfeder (9) bestimmt, bis zu welchem Drehmoment die Verzahnungen (21) der Rastbuchsen (3a, 3b) und Mitnehmerbüchsen (4, 5) im Eingriff bleiben, bevor sie durch Verdrehen gegeneinander die Weiterleitung des Drehmomentes auf den Schaft (1) unterbrechen.

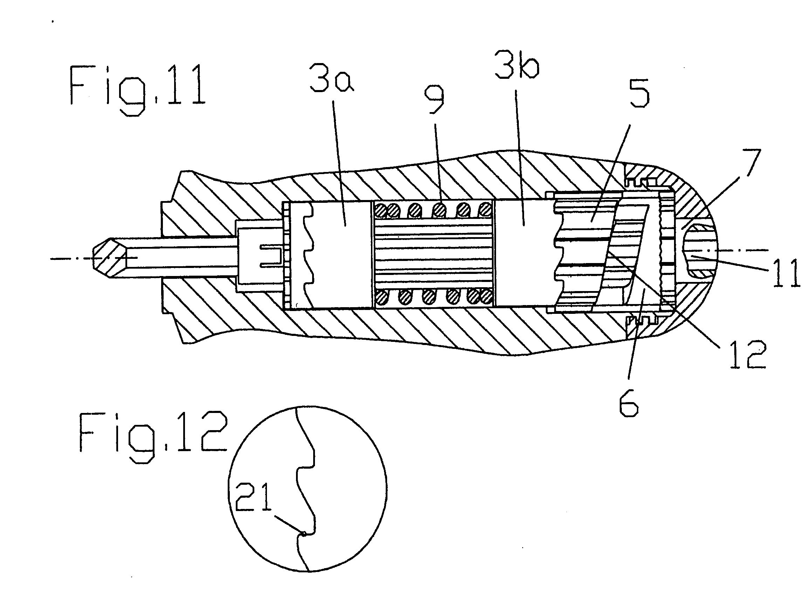

Fig. 11 zeigt einen Längsschnitt durch das Schraubwerkzeug, bei dem die hinteren Kupplungsteile (3b, 5) durch Verdrehen der Verstellscheibe (6) weiter nach vorne verschoben sind und die Feder (9) dadurch zusammengepresst und stärker vorgespannt ist. Zu erkennen ist dabei der zwischen der Verstellscheibe (6), beziehungsweise deren niedriger Bereich der schräg ansteigenden Ringfläche (12) und der zweiten Mitnehmerbüchse (5) entstandene Spalt.
Durch Drehen der Verstellscheibe (6) kann also ein bestimmtes, auf den Schaft (1) zu übertragendes Drehmoment eingestellt werden. Durch eine Kalibrierung kann der einstellbare Drehmoment-Bereich ermittelt und an einer Skala (16, 17) am Griffende dargestellt werden. Durch Auswahl der Druckfeder (9), beziehungsweise deren Federkennlinie, kann der gewünschte mit anderer Kennlinie ausgetauscht und anschließend die Mechanik, Kappe und Griff wieder montiert werden. Wie Fig. 2 zeigt, dient die Verstellscheibe (6) weiterhin als Lager für den Endabschnitt (10) der Zahnhülse (2), insbesondere als Axial-Lager. Die auftretenden Axialkräfte werden von der Verstellscheibe (6) über die Rastscheibe (8) in die Kappe (20) eingeleitet, beziehungsweise werden bei Benutzung des Schraubendrehers die von der Benutzerhand in den Griff (14) und die Kappe (20) eingeleitete Axialkraft in umgekehrter Richtung zum Schaft (1) geleitet.
Der Schaft (1) ist bei einer Ausführung gemäß Fig. 2 fest in die Zahnhülse (2) eingesetzt.

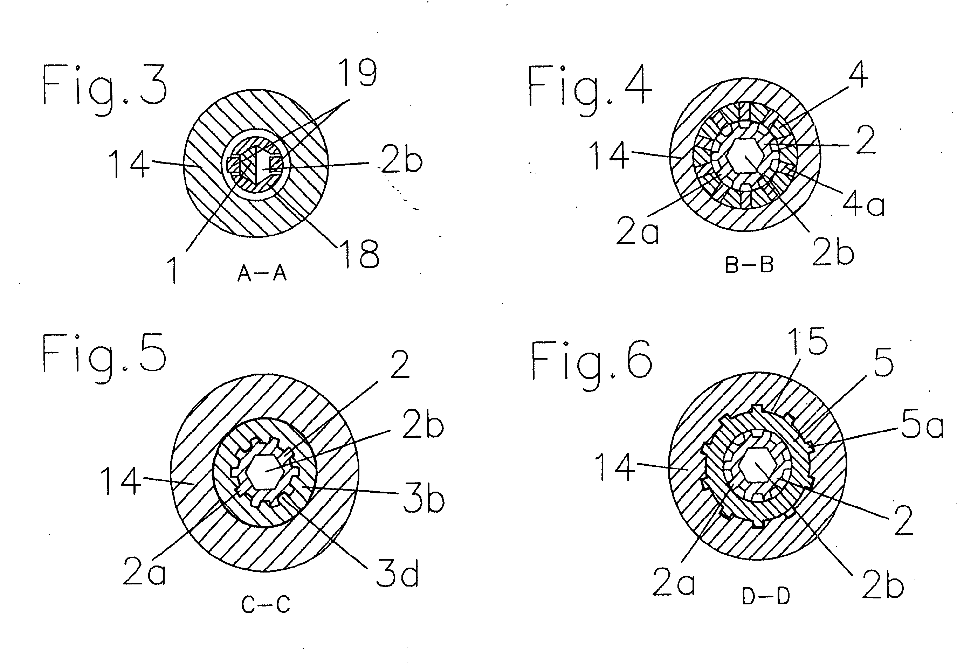

Figur 3 zeigt einen Querschnitt, entlang der Linie A-A in Fig. 1, nur durch den Bereich der Klemmvorrichtung (18, 19) bei der Ausführung, bei der der Schaft auswechselbar in die Zahnhülse eingesetzt ist. Er wird dabei in der vorgeformten, sich in Längsrichtung erstreckenden Höhlung in der Zahnhülse drehfest geführt und durch eine Klemmeinrichtung (18) am vorderen Ende der Zahnhülse (2) festgehalten. Die Klemmeinrichtung (18) ist einstückig mit der Zahnhülse als zylindrischer Ansatz ausgebildet und weist eine Federzunge oder einen Federsattel (19) auf, welche / welcher nach innen in die Höhlung in der Zahnhülse vorsteht und beim Einstecken des Schaftes in die Höhlung federnd radial ausgelenkt wird. Die Federspannung der ausgelenkten Zunge oder des Sattels hält den Schaft in seiner axialen Lage fest.
Die Klemmeinrichtung (18) kann auch zwischen den beiden Kupplungen angeordnet sein, abgedeckt durch die Feder (9). Das Profil der Zahnhülse (2) ist in dem Bereich der Klemmeinrichtung nicht durchgeführt, die Zahnhülse an dieser Stelle im Durchmesser etwas vergrößert. Eine alternative Ausführung der Klemmeinrichtung kann aus einer Band-Ringfeder und einer Kugel bestehen, wobei die Kugel in einer Radialbohrung in der Zahnhülse sitzt, etwas in die Höhlung (2b) der Zahnhülse hineinragt und von außen durch die Federkraft der Band- Ringfeder beanschlagt wird. Wird ein Schaft (1) in die Höhlung eingeführt, wird die Kugel nach außen radial verdrängt und die Band- Ringfeder wirkt mit einer Klemmkraft auf den Schaft.

In Fig. 4, dem Schnitt B-B sind zu erkennen: Die Stirnverzahnung (4a), mit der die erste Mitnehmerbüchse (4), in den Griff (14) eingreift und die Mitnehmerbüchse undrehbar mit ihm verbindet, außerdem die in der der ersten Mitnehmerbüchse (4) drehbar gelagerte Zahnhülse (2) mit ihrer Außenverzahnung (2a) und der Höhlung (2b) zur Aufnahme eines Sechskantschaftes (1).

In Fig. 5 dem Schnitt C-C, ist zu erkennen, wie die zweite Rastbuchse (3b) mit ihrer zylindrischen Umfangsfläche in der Höhlung des Griffes (14) drehbar gelagert und abgestützt ist - ebenso wie die erste Rastbuchse (3a) - und über eine Innenverzahnung (3d) mit der Außenverzahnung (2a) der Zahnhülse (2) verbunden ist.

In Fig. 6, dem Schnitt D-D, ist zu erkennen, dass die zweite Mitnehmerbüchse (5) über die Außenverzahnung (5a) mit der Innenverzahnung (15) des Griffes (14) verbunden, die Zahnhülse (2) ist in der Bohrung der zweiten Mitnehmerbüchse (5) drehbar gelagert.

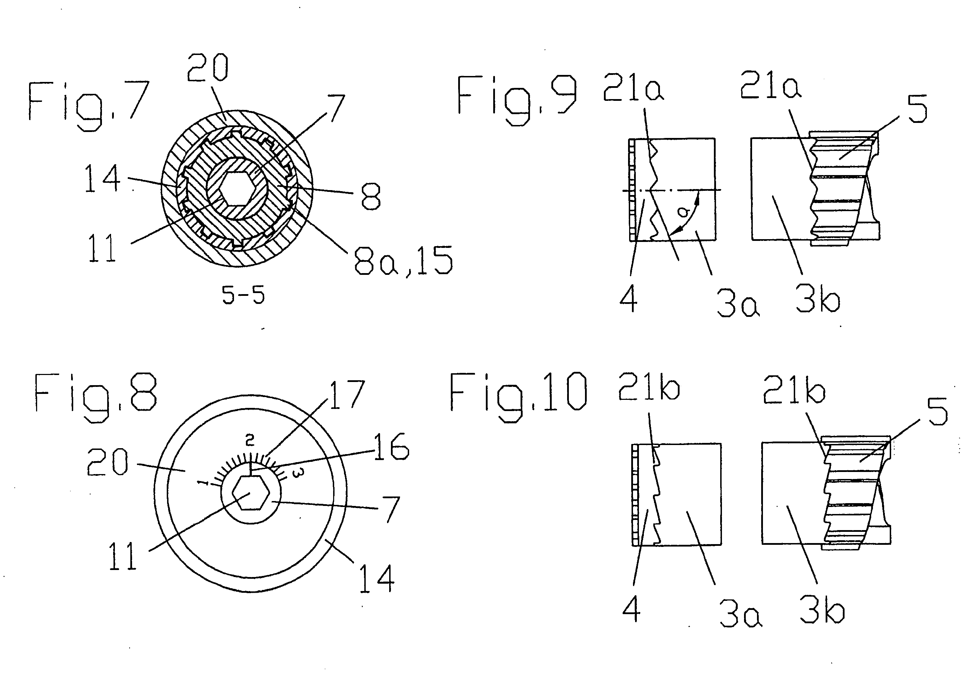

Fig. 7, der Schnitt E-E, zeigt, wie die Rastscheibe (8) über die Außenverzahnung (8a) undrehbar mit dem Griff (14) verbunden ist und der Zapfen (7) mit dem Sechskant- Innenprofil (11) drehbar in der Rastscheibe (8) gelagert ist.

In Fig. 8 ist die Markierung (16) am Zapfen (7) und die Skalierung (17) an der Kappe (20) als Prinzip dargestellt. Am Schraubwerkzeug wird zweckmäßigerweise in die Kappe eine Skalenscheibe eingelassen oder in anderer Weise befestigt sein, an der der einstellbare Drehmomentbereich unmittelbar angezeigt ist. Wie zuvor ausgeführt, hängt er von der Federkennlinie der eingesetzten Feder (9) ab und wird durch eine Kalibrierung ermittelt. Die Konstruktion von Verstellscheibe (6) und zweiter Mitnehmerhülse (5) - mit den schräg ansteigenden Ringflächen (12) ermöglicht eine sehr genaue Kalibrierung.
Der Steigungswinkel der Ringfläche (12) an der Mitnehmerhülse (5) und Verstellscheibe (6) bestimmt, in welchem Maße sich das übertragbare Drehmoment bei einem bestimmten Verstellwinkel der Verstellscheibe (6) verändert. Ist der Steigungswinkel klein, werden bei einem bestimmten Verstellwinkel die zweite Mitnehmerhülse (5) und die zweite Rastbuchse (3b) nur um eine geringe Wegstrecke verschoben und damit die Feder (9) um diese Wegstrecke in ihrer Länge verändert, entsprechend gering ist die Veränderung der Feder-Vorspannung und des übertragbaren Drehmomentes.
Für den Gebrauch ist es zweckmäßig, den jeweiligen Drehmomentbereich so zu unterteilen, dass Zwischenwerte in zweckmäßiger Stufung eingestellt werden können, zum Beispiel in Stufungen von 0,1 Nm oder 0,2 Nm. Im Zusammenwirken mit einer vorgewählten Feder geschieht dies konstruktiv durch die Wahl der Teilung der Verzahnung (6a), die die Verstellscheibe (6) mit der Rastscheibe (8) verbindet.
Entsprechend dieser Zahnteilung verändert sich stufenweise beim Verdrehen der Verstellscheibe (6) die Vorspannung der Feder (9) und damit das übertragbare Drehmoment bis zum Auslösen der Rastbuchsen (3a, 3b).

Die Figuren 9 und 10 zeigen die Kupplungen mit unterschiedlichen Ausführungen (21a, 21b) der Zahnformen. Durch die Zahnform kann der Drehmomentbereich ebenfalls beeinflusst werden. Ist der Flankenwinkel (α) bezogen auf die Drehachse der Kupplung, groß, so werden die Zähne bei kleinerem Drehmoment aneinander vorbeigleiten als wenn der Winkel α klein ist. Ist der Flankenwinkel 0°, wie in Fig. 10 in Drehrichtung nach links, so löst die Kupplung in dieser Drehrichtung nicht mehr aus. Außerdem wird, bei geringeren Losdreh- Widerstand der Schraube, ein Rückdrehen der Schraube vermieden.

Die Figuren 11 und 12 zeigen eine genauere Form des anschließen wellenförmigen Zahnprofiles der Verzahnung (21), die in den Figuren 1, 2, 9, 10 und 11 nur im Prinzip dargestellt ist.

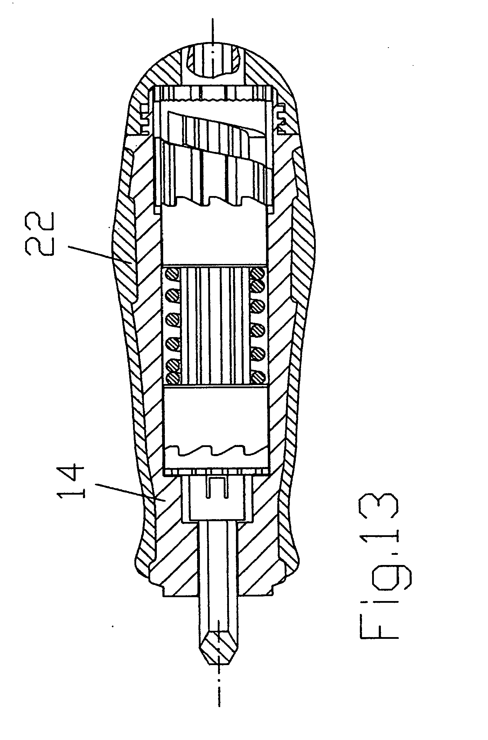

Die Anordnung von zwei Kupplungen und die übrige Konstruktionsausführung gemäß der Erfindung ermöglichen, höhere Drehmomente zu übertragen, als wenn nur eine Kupplung vorhanden wäre, weil die spezifische Belastung der Bauteile geringer ist, insbesondere der in Eingriff befindlichen Formelemente. Dazu trägt bei, dass das vom Griff auf den Schaft zu übertragene Drehmoment an zwei Stellen in die Übertragungsvorrichtung eingeleitet wird, nämlich über die Stirnverzahnung (4a) der ersten Mitnehmerbüchse (4) und über die Verzahnung (5a) der zweiten Mitnehmerbüchse (5) beziehungsweise der Innenverzahnung (15) des Griffes (14). Die Belastung einer Übertragungsstelle für das Drehmoment beziehungsweise einer Kupplung halbiert sich also im Vergleich zu einer solchen Vorrichtung mit nur einer Kupplung.
Es ist deshalb möglich, alle Bauteile bis auf den Schaft (1), die Feder (9) und die Scheiben (13) aus Kunststoff geeigneter Typen im Spritzgießverfahren herzustellen.
Als geeignet Kunststoff-Typen wurden Polyamid (PA) und Polyoxymethylen (POM) gefunden, insbesondere als Paarung bei solchen Teilen, die gleitend zusammenwirken. Als besonders vorteilhaft wurde ein Paarung gefunden, bei der POM mit Teflon kompoundiert ist. Dadurch wird die Reibung der aufeinander gleitenden Flächen zusätzlich vermindert, insbesondere an der Verzahnung (21) von Rast- und Mitnehmerbüchse. Hierdurch werden wiederum Einflüsse der Reibung auf das Auslöse- Drehmoment vermindert und die Genauigkeit der Vorrichtung weiter erhöht.
Schraubwerkzeuge mit einer Vorrichtung zur Begrenzung eines vorgewählten Drehmomentes werden jedoch auch in der orthopädischen Chirurgie benutzt und es ist erforderlich, diese nach Gebrauch zu sterilisieren. In Trokkenluft - Sterilisiergeräten wird eine Temperatur von 180°C erreicht. Bei dieser Temperatur soll keine bleibende Formänderung an den Teilen des Schraubwerkzeuges auftreten. In einer Sonderausführung wird das Schraubwerkzeug deshalb aus Kunststoffen hergestellt, deren Formstabilität bis zu einer Temperatur von ca. 220°C noch gewährleistet ist.
Teile, wie die Zahnhülse (2) die Rastbüchsen (3a, 3b) die Mitnehmerbüchsen (4, 5) die Verstellscheibe (6) die Rastscheibe (8) oder einzelne dieser Teile können alternativ auch aus Metall hergestellt sein, zum Beispiel als Sinterteile in einer Metallzusammensetzung und/oder Metall-Paarung, die gute Gleiteigenschaften gegeneinander aufweisen, oder auch aus Stahl, spanend oder durch Pressen geformt.
Auch der Griff (14) und die Kappe (20) können aus Metall etwa aus Aluminium-Druckguss hergestellt sein, und eine durch Eloxieren veredelte Oberfläche aufweisen. Schließlich können Griff, Kappe und andere, insbesondere statisch belastete Teil, aus Edelstahl gefertigt sein, etwa im Wachs- Ausschmelzverfahren oder aus Pulvermetall im Spritzgieß-Sinterverfahren. Die Metallausführung kommt in Betracht, wenn bei gleichen Abmessungen wie bei der Kunststoffausführung höhere Drehmomente übertragen werden sollen oder, bei Verwendung in der orthopädischen Chirurgie, eine uneingeschränkte Sterilisierbarkeit gegeben sein soll. Da bei einer Ausführung des Griffes aus Metall zur Gewichtsminderung die Wanddicke nach den Anforderungen an eine ausreichende Haltbarkeit ausgelegt wird, weniger nach den Anforderungen an eine gute Haptik, ist in einer Ausführungsvariante vorgesehen auf den Grundkörper des Griffes, der im Querschnitt eine unrunde Außenkontur aufweist, eine Griffhülse aus vorzugsweise elastischem Kunststoff aufzustecken, deren Außenform nach Anforderungen der Ergonomie gestaltet ist. Diese Griffhülse wird nach Gebrauch vom Grundkörper abgezogen und entsorgt, insbesondere, wenn sie aus einem elastischen, weniger temperaturbeständigen Kunststoff hergestellt ist und nicht sterilisiert werden kann. Diese Ausführungsvariante ist in Fig. 13 beispielhaft dargestellt. Darin sind (14) der Griff aus Metall, (22) die Griffhülse aus elastischem Kunststoff.

Vorteilhaft ausgeführt ist auch die Einstelleinrichtung für das zu übertragene Drehmoment. Das Zusammenwirken der schräg ansteigenden Ringflächen (12) und die feine Verzahnung (6a) an der Rastscheibe (8) und an der Verstellscheibe (6) ermöglichen kleine Verstellschritte, somit eine genaue Einstellung der Vorspannung der Feder (9) und somit des AuslöseDrehmomentes. Die Vorspannung der Feder hält die Rastscheibe in der eingestellten Lage fest. Durch die Wahl eines kleinen Steigungswinkels der Ringfläche (12) können die übertragbaren Drehmomente besonders fein und genau eingestellt werden. Ein so ausgestattetes Schraubwerkzeug kann deshalb auch zum Kalibrieren anderer Drehmoment anzeigenden Geräte benutzt werden oder dort, wo es darauf ankommt, ein Grenzdrehmoment besonders genau einzuhalten.

Durch die achszentrische Anordnung von zwei Kupplungen bestehend aus den Rastbuchsen (3a, 3b) und den damit zusammenwirkenden Mitnehmerbüchsen (4, 5) wird die Vorspannungskraft der Feder (9) gleichmäßig auf beide Kupplungen verteilt, und auf der einen Seite über die erste Mitnehmerbüchse (4), auf der anderen Seite über die Verstellscheibe (6) und Rastscheibe (8) in den Griff (14) beziehungsweise die Kappe (20) abgeleitet, die Verspannungskraft wirkt nicht mit axialer Belastung auf die Zahnhülse und deren Axial-Lager. Es wird vermieden, dass durch eine auf die die axiale Lagerung der Zahnhülse (2) wirkende Vorspannungskraft die Reibung in deren axialen Lagerung erhöht und dadurch die übertragenden Drehmomente vom Griff zum Schaft beeinflußt werden, was zu Ungenauigkeiten beim Auslöse-Drehmoment führen würde.
Vorteilhaft ist bei der Konstruktion gemäß der Erfindung, dass die Montage aller Bauteile auf einfache Weise möglich, so dass ein Drehmoment-Schraubwerkzeug gemäß der Erfindung kostengünstig herstellbar ist, eine hohe Funktionsgenauigkeit und lange Lebensdauer hat.

## Patentansprüche

1. Schraubwerkzeug mit einer in der Höhlung eines Griffes angeordneten Vorrichtung zur Begrenzung des übertragenen Drehmomentes, bestehend aus dem Griff als Antriebsteil, einem in der Vorrichtung gelagerten Schaft als Abtriebsteil und zwischen dem Griff und dem Schaft angeordneten Kupplungselementen, die bei Überschreiten eines vorgewählten Drehmomentes die Weiterleitung des Drehmomentes vom Antriebsteil zum Abtriebsteil unterbrechen, **dadurch gekennzeichnet, dass** zwei Kupplungen, jeweils bestehend aus einer ersten und zweiten Rastbuchse (3a, 3b) und einer ersten und zweiten Mitnehmerbüchse (4, 5) und eine Druckfeder (9) mit Vorspannung zwischen den Kupplungen auf einer Zahnhülse (2) angeordnet sind, die einander zugewandten Stirnseiten von Rastbuchsen (3a, 3b) und Mitnehmerbüchsen (4, 5) ineinandergreifende Verzahnungen (21) aufweisen, die Mitnehmerbüchsen (4, 5) formschlüssig und drehfest mit dem Griff (14) verbunden und drehbar auf der Zahnhülse gelagert sind, während die Rastbuchsen (3a, 3b) mit einer Innenverzahnung (3d) in eine formgleiche Außenverzahnung (2a) der Zahnhülse (2) eingreifen und auf ihr drehfest und axial verschiebbar sind.

2. Schraubwerkzeug mit einer Vorrichtung zur Begrenzung des übertragenen Drehmomentes nach Anspruch 1 **dadurch gekennzeichnet, dass** die Mitnehmerbüchsen (4, 5) eine runde Axialbohrung aufweisen, in der die Zahnhülse (2) drehbar geführt ist.

3. Schraubwerkzeug mit einer Vorrichtung zur Begrenzung des übertragenen Drehmomentes nach Anspruch 1 und 2 **dadurch gekennzeichnet, dass** die Rastbuchsen (3a, 3b) mit ihrer zylindrischen Umfangsfläche in der sie zumindest auf einem Teil der Längserstreckung umgebenden zylindrischen Höhlung des Griffes (14) geführt und radial gestützt sind.

4. Schraubwerkzeug mit einer Vorrichtung zur Begrenzung des übertragenen Drehmomentes nach Anspruch 1 bis 3 **dadurch gekennzeichnet, dass** die erste Mitnehmerbüchse (4) an ihrer von der ersten Rastbuchse (3a) abgewendeten Stirnseite durch eine Stirnverzahnung (4a) formschlüssig undrehbar mit dem Griff (14) verbunden ist.

5. Schraubwerkzeug mit einer Vorrichtung zur Begrenzung des übertragenen Drehmomentes nach Anspruch 1 bis 4 **dadurch gekennzeichnet, dass** die zweite Mitnehmerbüchse (5) drehbar und verschiebbar auf der Zahnhülse (2) gelagert ist und eine sich über die Umfangsfläche erstrekkende Verzahnung (5a) aufweist, in die eine formgleiche Innenverzahnung (15) eingreift, welche in diesem Bereich in der Höhlung des Griffes (14) eingeformt ist.

6. Schraubwerkzeug mit einer Vorrichtung zur Begrenzung des übertragenen Drehmomentes nach Anspruch 1 bis 5 **dadurch gekennzeichnet, dass** die zweite Mitnehmerbüchse (5) an ihrer von der zweiten Rastbuchse (3b) abgewendeten Stirnseite eine in Umfangsrichtung schräg ansteigende Ringfläche (12) aufweist.

7. Schraubwerkzeug mit einer Vorrichtung zur Begrenzung des übertragenen Drehmomentes nach Anspruch 1 bis 6 **dadurch gekennzeichnet, dass** eine Verstellscheibe (6) an einer Stirnseite ebenfalls eine in Umfangsrichtung schräg ansteigende Ringfläche (12) aufweist, die an der formgleichen Ringfläche der zweiten Mitnehmerbüchse (5) anliegt, und die Verstellscheibe auf der anderen Stirnseite eine feine Verzahnung (6a) aufweist.

8. Schraubwerkzeug mit einer Vorrichtung zur Begrenzung des übertragenen Drehmomentes nach Anspruch 1 bis 7 **dadurch gekennzeichnet, dass** die Verstellscheibe (6) mit einem Zapfen (7) eine Rastscheibe (8) und die Kappe (20) des Griffes (14) axial durchdringt, in beiden drehbar gelagert ist und in den Zapfen von seinem Ende her eine Höhlung (11) zum Eingriff eines Verstellschlüssels eingeformt ist.

9. Schraubwerkzeug mit einer Vorrichtung zur Begrenzung des übertragenen Drehmomentes nach Anspruch 1 bis 8 **dadurch gekennzeichnet, dass** die Rastscheibe (8) an ihrer der Verstellscheibe (6) zugewandten Stirnseite ebenfalls mit einer feinen Verzahnung (6a) versehen ist und an ihrem Umfang mit einer Verzahnung (8a), mit der sie in die formgleiche Innenverzahnung (15) in der Höhlung des Griffes (14) eingreift.

10. Schraubwerkzeug mit einer Vorrichtung zur Begrenzung des übertragenen Drehmomentes nach Anspruch 1 bis 9 **dadurch gekennzeichnet, dass** die Teilung der Verzahnung (6a) an der Verstellscheibe (6) und Rastscheibe (8) so gewählt ist, dass für einen bestimmten vorgewählten Drehmomentbereich Zwischenwerte in zweckmäßiger gleicher Abstufung von Bruchteilen einer Drehmoment-Einheit eingestellt werden können.

11. Schraubwerkzeug mit einer Vorrichtung zur Begrenzung des übertragenen Drehmomentes nach Anspruch 1 bis 10 **dadurch gekennzeichnet, dass** die Oberfläche am Ende des Zapfens (7) des Verstellelementes (6) eine Markierung (16) oder Skalierung aufweist, ebenso die Oberfläche der Kappe (20) um den Zapfen (7) herum eine Markierung oder Skalierung (17) und die Stellung der beiden Markierungen oder Skalierungen zueinander die Höhe der Vorspannung der Druckfeder (9) und entsprechend das eingestellte maximal übertragbare Drehmoment anzeigen.

12. Schraubwerkzeug mit einer Vorrichtung zur Begrenzung des übertragenen Drehmomentes nach Anspruch 1 bis 11 **dadurch gekennzeichnet, dass** das vorgewählte maximal zu übertragene Drehmoment in der Weise eingestellt wird, dass durch Verdrehen der Verstellscheibe (6) deren schräg ansteigende Ringfläche (12) mit der schräg ansteigenden Ringfläche (12) der zweiten Mitnehmerhülse (5) zusammenwirkend eine axiale Verschiebung der zweiten Mitnehmerbüchse (5) und der zweiten Rastbuchse (3b) bewirkt und **dadurch** die Vorspannung der Druckfeder (9) verändert wird.

13. Schraubwerkzeug mit einer Vorrichtung zur Begrenzung des übertragenen Drehmomentes nach Anspruch 1 bis 12 **dadurch gekennzeichnet, dass** beim Einstellen der Vorspannung der Feder (9), beziehungsweise des gewählten maximalen Drehmomentes, durch Drehen der Verstellscheibe (6) die Verzahnungen (6a) in der Verstellscheibe (6) und in der Rastscheibe (8) unter Axialbewegungen der zweiten Rastbuchse (3b) und zweiten Mitnehmerbüchse (5) gegen den Druck der Feder (9) aneinander vorbeigleiten und in der durch die Drehung vorgegebenen, an der Skalierung angezeigten gewählten Stellung, wieder miteinander in formschlüssigen Eingriff kommen.

14. Schraubwerkzeug mit einer Vorrichtung zur Begrenzung des übertragenen Drehmomentes nach Anspruch 1 bis 13 **dadurch gekennzeichnet, daß** die Verzahnung ein im wesentlichen dreieckförmiges Profil (21a) aufweist, bei dem der Flankenwinkel α der Zähne - bezogen auf die Drehachse - in Richtung der Drehung beim Anziehen von Schrauben - nach rechts - größer ist als in Richtung der Drehung beim Lösen von Schrauben - nach links.

15. Schraubwerkzeug mit einer Vorrichtung zur Begrenzung des übertragenen Drehmomentes nach Anspruch 1 bis 14 **dadurch gekennzeichnet, daß** der Flankenwinkel α der Zähne - bezogen auf die Drehachse - in Drehrichtung zum Anziehen der Schrauben bei einem Schraubwerkzeug für einen Bereich kleiner zu übertragender Drehmomente größer ist als bei einem Schraubwerkzeug für einen Bereich höherer zu übertragender Drehmomente.

16. Schraubwerkzeug mit einer Vorrichtung zur Begrenzung des übertragenen Drehmomentes nach Anspruch 1 bis 13 **dadurch gekennzeichnet, daß** die Verzahnung ein im wesentlichen dreieckförmiges Profil (21b) aufweist, bei dem der Flankenwinkel der Zähne in Drehrichtung zum Lösen von Schrauben - nach links - 0° , bezogen auf die Drehachse, beträgt.

17. Schraubwerkzeug mit einer Vorrichtung zur Begrenzung des übertragenen Drehmomentes nach Anspruch 1 bis 16 **dadurch gekennzeichnet, dass** die Verzahnungen (21) an den Zahnspitzen abgerundet oder abgeflacht sind

18. Schraubwerkzeug mit einer Vorrichtung zur Begrenzung des übertragenen Drehmomentes nach Anspruch 1 bis 17 **dadurch gekennzeichnet, dass** die Verzahnungen (21) wellenförmige Zahnprofile aufweisen.

19. Schraubwerkzeug mit einer Vorrichtung zur Begrenzung des übertragenen Drehmomentes nach Anspruch 1 bis 18 **dadurch gekennzeichnet, dass** die Verzahnungen (2a, 5a, 15) Rechteck-Verzahnungen sind.

20. Schraubwerkzeug mit einer Vorrichtung zur Begrenzung des übertragenen Drehmomentes nach Anspruch 1 bis 19 **dadurch gekennzeichnet, dass** der Griff (14) und die Kappe (20) miteinander verschraubt sind.

21. Schraubwerkzeug mit einer Vorrichtung zur Begrenzung des übertragenen Drehmomentes nach Anspruch 1 bis 20 **dadurch gekennzeichnet, dass** der Griff (14) und die Kappe (20) unlösbar verbunden sind.

22. Schraubwerkzeug mit einer Vorrichtung zur Begrenzung des übertragenen Drehmomentes nach Anspruch 1 bis 21 **dadurch gekennzeichnet, dass** die Zahnhülse (2), die Rastbuchsen (3a, 3b) die Mitnehmerbüchsen (4, 5), die Verstellscheibe (6), die Rastscheibe (8), die Feder (9), der Griff (14) und die Kappe (20) so zueinander angeordnet und in Eingriff sind, dass die durch die Druckfeder (9) bewirkte Vorspannungskraft die Zahnhülse (2) nicht axial belastet.

23. Schraubwerkzeug mit einer Vorrichtung zur Begrenzung des übertragenen Drehmomentes nach Anspruch 1 bis 22 **dadurch gekennzeichnet, dass** zwischen der Druckfeder (9) und den Rastbuchsen (3a, 3b) Stahlscheiben (13) angeordnet sind.

24. Schraubwerkzeug mit einer Vorrichtung zur Begrenzung des übertrage= nen Drehmomentes nach Anspruch 1 bis 23 **dadurch gekennzeichnet, daß** alle Drehmomente in dem durch eine Feder (9) vorbestimmten Bereich durch Verdrehen der Verstellscheibe (6) in einem Winkelbereich von 0° bis 360° eingestellt werden können.

25. Schraubwerkzeug mit einer Vorrichtung zur Begrenzung des übertragenen Drehmomentes nach Anspruch 1 bis 24 **dadurch gekennzeichnet, daß** die Vorspannkraft der Feder (9) über die zweite Rastbuchse (3b) und die zweite Mitnehmerbüchse (5) die Verstellscheibe (6) und Rastscheibe (8) beaufschlagt.

26. Schraubwerkzeug mit einer Vorrichtung zur Begrenzung des übertragenen Drehmomentes nach Anspruch 1 bis 25 **dadurch gekennzeichnet, dass** die Zahnhülse (2), die Rastbuchsen (3a, 3b), die Mitnehmerbüchsen (4, 5), die Verstellscheibe (6),die Rastscheibe (8), der Griff (14) und die Kappe (20) vorzugsweise aus Kunststoff geeigneter Typen im Spritzgießverfahren hergestellt sind.

27. Schraubwerkzeug mit einer Vorrichtung zur Begrenzung des übertragenen Drehmomentes nach Anspruch 1 bis 26 **dadurch gekennzeichnet, dass** bei gleitend zusammenwirkenden Teilen jeweils ein Teil aus Polyamid und das andere Teil aus Polyoxymethylen hergestellt ist, wobei zumindest einer dieser Kunststoffe mit Teflon compoundiert ist.

28. Schraubwerkzeug mit einer Vorrichtung zur Begrenzung des übertragenen Drehmomentes nach Anspruch 1 bis 27 **dadurch gekennzeichnet, dass** die verwendeten Kunststoffe im Temperaturbereich bis etwa 220°C formstabil sind.

29. Schraubwerkzeug mit einer Vorrichtung zur Begrenzung des übertragenen Drehmomentes nach Anspruch 1 bis 27 **dadurch gekennzeichnet, dass** die Zahnhülse (2) die Rastbuchsen (3a, 3b), die Mitnehmerbüchsen (4, 5), die Verstellscheibe (6), die Rastscheibe (8), der Griff (14), die Kappe (20), oder einzelne dieser Teile, aus Metall hergestellt sind.

30. Schraubwerkzeug mit einer Vorrichtung zur Begrenzung des übertragenen Drehmomentes nach Anspruch 1 bis 25 und 29 **dadurch gekennzeichnet, dass** die äußere Querschnittskontur des Griffes (14) aus Metall unrund ist und eine ergonomisch geformte Griffhülse (22) aus Kunststoff auswechselbar auf den Griff (14) aufgesteckt ist.

31. Schraubwerkzeug mit einer Vorrichtung zur Begrenzung des übertragenen Drehmomentes nach Anspruch 1 bis 30 **dadurch gekennzeichnet, dass** der Schaft (1) fest in der Zahnhülse (2) eingesetzt ist.

32. Schraubwerkzeug mit einer Vorrichtung zur Begrenzung des übertragenen Drehmomentes nach Anspruch 1 bis 30 **dadurch gekennzeichnet, dass** der Schaft (1) auswechselbar in der Zahnhülse (2) eingesetzt ist und durch eine an die Zahnhülse einstückig angeformte Klemmvorrichtung (18, 19) axial gehalten wird.

33. Schraubwerkzeug mit einer Vorrichtung zur Begrenzung des übertragenen Drehmomentes nach Anspruch 1 und 32 **dadurch gekennzeichnet, dass** der Schaft (1) durch ein unrundes Querschnittsprofil oder anders gestaltete Mitnehmerelemente undrehbar mit der Zahnhülse (2) verbunden ist, sich der Schaft (1) bis kurz vor das hintere Ende der Zahnhülse erstreckt und dort axial abgestützt ist.

## Claims

1. A screwing tool having, in a hollow of a handle, a device for limiting transmitted torque, consisting of the handle as a driving means, of a shaft as a driven means lodged in the device and of coupling components arranged between the handle and the shaft, which coupling components are adapted to interrupt the transmission of torque from the driving means to the driven means when a selected torque value is exceeded, wherein two couplings each of which comprises first and second locking sockets (3a, 3b) as well as first and second gear sockets (4, 5) and a compression spring (9) are arranged with pretension on a gear sleeve (2), the opposing front faces of the locking sockets (3a, 3b) and of the gear sockets (4, 5) having gear teeth (21), the gear sockets (4, 5) being positively attached to the handle (14) for turning therewith and being rotatably supported on the gear sleeve (2) whereas the locking sockets (3a, 3b) have an inner gearing (3d) that engages an outer gearing (2a) of matching shape at the gear sleeve (2) on which they are arranged for axial shifting and turning therewith.

2. Screwing tool having a device for limiting transmitted torque according to claim 1, wherein the gear sockets (4, 5) comprise a round axial bore in which the gear sleeve (2) is rotatably guided.

3. Screwing tool having a device for limiting transmitted torque according to claims 1 and 2, wherein the cylindrical peripheral surfaces of the locking sockets (3a, 3b) are guided and radially supported by cylindrical hollows in the handle (14) which enclose said sockets (3a, 3b) along at least a part of their longitudinal extensions.

4. Screwing tool having a device for limiting transmitted torque according to claims 1 to 3, wherein the first gear socket (4) is, at its face pointing off the first locking socket (3a), positively and unturnably connected to the handle (14).

5. Screwing tool having a device for limiting transmitted torque according to claims 1 to 4, wherein the second gear socket (5) is rotatably and shiftably supported on the gear sleeve (2) and includes gear teeth (5a) that extend over the peripheral surface and that are engaged by gear teeth (15) of matching shape formed in this area of the hollow of the handle (14).

6. Screwing tool having a device for limiting transmitted torque according to claims 1 to 5, wherein the second gear socket (5) comprises, at its face pointing off the second locking socket (3b), an oblique annular surface (12) that ascends in a circumferential direction.

7. Screwing tool having a device for limiting transmitted torque according to claims 1 to 6, wherein at a front face of an adjusting disc (6), there is also an oblique annular surface (12) that ascends in a circumferential direction and engages the oblique annular surface of matching shape of the second gear socket (5), which adjusting disc (6) includes, at its opposite front face, fine gear teeth (6a).

8. Screwing tool having a device for limiting transmitted torque according to claims 1 to 7, wherein a pin (7) of the adjusting disc (6) axially extends through a locking disc (8) and through a cap (20) of the handle (14), being rotatably supported in these two elements (8, 20), and wherein a hollow (11) to be engaged by an adjusting key is formed inwardly at an end of the pin (7).

9. Screwing tool having a device for limiting transmitted torque according to claims 1 to 8, wherein the locking disc (8) also comprises fine gear teeth (6a) at a front face pointing to the adjusting disc (6) and further comprises, at its periphery, a gearing (8a) that engages the matching inner gearing (15) in the hollow of the handle (14).

10. Screwing tool having a device for limiting transmitted torque according to claims 1 to 9, wherein the pitch of the gearing (6a) at the adjusting disc (6) and at the locking disc (8) is selected such that for a predetermined range of torques, intermediate torque values can be set, expediently by uniform steps of fractions of a torque unit.

11. Screwing tool having a device for limiting transmitted torque according to claims 1 to 10, wherein the surface at the end of the pin (7) of the adjusting component (6) and the surface of the cap (20) around the pin (7) each include a marking (16) or a scale and wherein the mutual positions of the two markings or scales indicate the amount of pretension of the compression spring (9) and thus the maximum torque set for transmission.

12. Screwing tool having a device for limiting transmitted torque according to claims 1 to 11, wherein the selected maximum torque to be transmitted is set in such manner that by turning the adjusting disc (6), its oblique ascending annular surface (12) will, in cooperation with the oblique ascending annular surface (12) of the second gear socket (5), cause axial shifting of the second gear socket (5) and of the second locking socket (3b) so as to alter the pretension of the compression spring (9).

13. Screwing tool having a device for limiting transmitted torque according to claims 1 to 12, wherein during the pretension setting of the compression spring (9) or the setting of the maximum torque to be transmitted, respectively, the gearings (6a) in the adjusting disc (6) and in the locking disc (8) will slidingly pass each other with axial movements of the second locking socket (3b) and of the second gear socket (5) against the bias of the spring (9), so as to again positively engage each other in a position that is given by the amount of rotation and is indicated at the scale.

14. Screwing tool having a device for limiting transmitted torque according to claims 1 to 13, wherein the gearings each comprise a substantially triangular tooth profile (21a), with the angle α of the tooth flanks - relative to the axes of the sockets - being larger in the direction of screw fastening (turning to the right) than in the direction of unscrewing (turning to the left).

15. Screwing tool having a device for limiting transmitted torque according to claims 1 to 14, wherein an angle α of the tooth flanks - relative to the axes of the sockets - in the direction of screw fastening is larger for a screwing tool designed to transmit a range of lower torques than for a screwing tool designed to transmit a range of higher torques.

16. Screwing tool having a device for limiting transmitted torque according to claims 1 to 13, wherein the gearings comprise a substantially triangular tooth profile (21b), with the angle of the tooth flanks being 0° relative to the axis of rotation (turning to the left).

17. Screwing tool having a device for limiting transmitted torque according to claims 1 to 16, wherein the teeth of the gearings (21) are rounded or flattened at their ridges.

18. Screwing tool having a device for limiting transmitted torque according to claims 1 to 17, wherein the teeth of the gearings (21) are of wave-like shape.

19. Screwing tool having a device for limiting transmitted torque according to claims 1 to 18, wherein the teeth of the gearings (2a, 5a, 15) have rectangular shapes.

20. Screwing tool having a device for limiting transmitted torque according to claims 1 to 19, wherein the handle (14) and the cap (20) are screw-connected.

21. Screwing tool having a device for limiting transmitted torque according to claims 1 to 20, wherein the handle (14) and the cap (20) are inseparably connected.

22. Screwing tool having a device for limiting transmitted torque according to claims 1 to 21, wherein the gear sleeve (2), the locking sockets (3a, 3b), the gear sockets (4, 5), the adjusting disc (6), the locking disc (8), the spring (9), the handle (14) and the cap (20) are mutually arranged and engaging in such manner that no axial load onto the gear sleeve (2) is exerted by the biassing force of the compression spring (9).

23. Screwing tool having a device for limiting transmitted torque according to claims 1 to 22, wherein steel discs are arranged between the compression spring (9) and the locking sockets (3a, 3b).

24. Screwing tool having a device for limiting transmitted torque according to claims 1 to 23, wherein within a range predetermined by a spring (9), any torques can be set by turning the adjusting disc (6) through an angular range from 0° to 360°.

25. Screwing tool having a device for limiting transmitted torque according to claims 1 to 24, wherein the biassing force of the spring (9) acts on the adjusting disc (6) and on the locking disc (8) via the second locking socket (3b) and the second gear socket (5).

26. Screwing tool having a device for limiting transmitted torque according to claims 1 to 25, wherein the gear sleeve (2), the locking sockets (3a, 3b), the gear sockets (4, 5), the adjusting disc (6), the locking disc (8), the handle (14) and the cap (20) are made by injection moulding, preferably of suitable plastic materials.

27. Screwing tool having a device for limiting transmitted torque according to claims 1 to 26, wherein of parts that are arranged for sliding along each other, one part is made of polyamide and the other part is made of polyoxymethylene, at least one of these plastic materials being compounded with Teflon.

28. Screwing tool having a device for limiting transmitted torque according to claims 1 to 27, wherein the plastic materials used are dimensionally stable up to about 220 C.

29. Screwing tool having a device for limiting transmitted torque according to claims 1 to 27, wherein the gear sleeve (2), the locking sockets (3a, 3b), the gear sockets (4, 5), the adjusting disc (6), the locking disc (8), the handle (14), the cap (20) or single ones of these component parts are made of metal.

30. Screwing tool having a device for limiting transmitted torque according to claims 1 to 25 and 29, wherein the outer cross section of the handle (14) has a non-circular contour and wherein an ergonomically shaped handle sleeve (22) made of plastic material is exchangeably mounted onto the handle (14).

31. Screwing tool having a device for limiting transmitted torque according to claims 1 to 30, wherein the shaft (1) is fixedly inserted into the gear sleeve (2).

32. Screwing tool having a device for limiting transmitted torque according to claims 1 to 30, wherein the shaft (1) is exchangeably inserted into the gear sleeve (2) and is axially held therein by clamping means (18, 19) integrally formed onto the gear sleeve (2).

33. Screwing tool having a device for limiting transmitted torque according to claims 1 and 32, wherein the shaft (1) is unturnably connected to the gear sleeve (2) by a profile of non-circular cross section or by gear components shaped otherwise and wherein the shaft (1) extends almost to the rear end of the gear sleeve (2) and is axially supported there.

## Revendications

1. Outil à visser avec dispositif limitateur de couple transmis disposé dans le creux d'une poignée et consistant en une poignée servant de partie d'entraînement, en une tige logée dans le dispositif et servant de partie entraînée, et en des éléments d'embrayage disposés entre la poignée et la tige qui coupent la transmission du couple de rotation de la partie d'entraînement vers la partie entraînée lorsqu'un couple présélectionné est dépassé, **caractérisé en ce que** deux embrayages, qui consistent respectivement en une première et une deuxième douille d'enclenchement (3a, 3b) et en une première et une deuxième boîte d'entraînement (4, 5), et un ressort de pression (9) sous prétension sont disposés entre les embrayages sur une douille dentée (2), **en ce que** les côtés frontaux des douilles d'enclenchement (3a, 3b) et des boîtes d'entraînement (4, 5) qui font face ont des dentures (21) s'engrenant les unes dans les autres, et **en ce que** les boîtes d'entraînement (4, 5) sont assemblées à la poignée (14), par engagement positif et de manière à résister à la rotation, et logées rotatives sur la douille dentée, alors que les douilles d'enclenchement (3a, 3b) s'engrènent avec une denture intérieure (3d) dans une denture extérieure de même forme (2a) de la douille dentée (2) et qu'elles sont logées sur cette dernière d'une manière rotativement fixe et axialement mobile.

2. Outil à visser avec dispositif limitateur de couple transmis selon la revendication 1, **caractérisé en ce que** les boîtes d'entraînement (4, 5) ont une perforation axiale ronde, dans laquelle la douille dentée (2) est guidée d' une manière rotative.

3. Outil à visser avec dispositif limitateur de couple transmis selon la revendication 1 et 2, **caractérisé en ce que** les douilles d'enclenchement (3a, 3b), avec leurs surfaces circonférentielles cylindriques, sont guidées et s' appuyent d' une manière radiale dans le creux cylindrique de la poignée (14) qui les entourent au moins sur une partie de l'étendue longitidinale.

4. Outil à visser avec dispositif limitateur de couple transmis selon la revendication 1 à 3, **caractérisé en ce que** la première douille d'entraînement (4), sur son côté frontal détourné de la première douille d'enclenchement (3a), est assemblée à l'aide d'une denture frontale (4a) à la poignée (14) par engagement positif et de manière non-rotative.

5. Outil à visser avec dispositif limitateur de couple transmis selon la revendication 1 à 4, **caractérisé en ce que** la deuxième boîte d'entraînement (5) est logée d' une manière rotative et coulissable sur la douille dentée (2), et **en ce qu'**elle dispose d'une denture (5a) qui s'étend sur la surface circonférentielle et dans laquelle qui s'engrène une denture intérieure de même forme (15), laquelle a été façonnée dans cette partie du creux de la poignée (14).

6. Outil à visser avec dispositif limitateur de couple transmis selon la revendication 1 à 5, **caractérisé en ce que** la deuxième boîte d'entraînement (5) a sur son côté frontal détourné de la deuxième douille d'enclenchement (3b) une surface annulaire (12) montant obliquement en direction circonférentielle.

7. Outil à visser avec dispositif limitateur de couple transmis selon la revendication 1 à 6, **caractérisé en ce qu'**un disque de réglage (6) sur un côté frontal dispose également d'une surface annulaire (12) montant obliquement en direction circonférentielle, laquelle se porte bien avec la surface annulaire de même forme de la deuxième boîte d'entraînement (5), et **en ce que** sur l'autre côté frontal, le disque de réglage dispose d'une fine denture (6a).

8. Outil à visser avec dispositif limitateur de couple transmis selon la revendication 1 à 7, **caractérisé en ce qu'**un tourillon (7), du disque de réglage (6) traverse axialement un disque d'enclenchement (8) et le capuchon (20) de la poignée (14), **en ce qu'**il est logé d' une manière rotative dans ces deux derniers, et **en ce qu'**un creux (11) a été façonné dans l'extrémité du tourillon pour donner prise à une clé de réglage.

9. Outil à visser avec dispositif limitateur de couple transmis selon la revendication 1 à 8, **caractérisé en ce que** le disque d'enclenchement (8) est également pourvu d'une fine denture (6a) sur son côté frontal tourné vers le disque de réglage (6), et **en ce qu'**il est pourvu sur sa circonférence d'une denture (8a) avec laquelle il s'engrène dans la denture intérieure de même forme (15) dans le creux de la poignée (14).

10. Outil à visser avec dispositif limitateur de couple transmis selon la revendication 1 à 9, **caractérisé en ce que** le pas primitif de la denture (6a) sur le disque de réglage (6) et sur le disque d'enclenchment (8) a été choisi de telle manière, que pour un certain domaine présélectionné du couple, il est possible de choisir des valeurs intermédiaires graduées utilement par fractions identiques d'une unité de couple.

11. Outil à visser avec dispositif limitateur de couple transmis selon la revendication 1 à 10, **caractérisé en ce que** la surface à l'extrémité du tourillon (7) de l'élément de réglage (6) dispose d'un repère (16) ou d'une graduation, **en ce que** la surface du capuchon (20) autour du tourillon (7) dispose également d'un repère ou d'une graduation (17), et **en ce que** la position de ces deux graduations ou repères l'une par rapport à l'autre indique le degré de prétension du ressort de pression (9), et donc le couple de rotation sélectionné pouvant être transmis tout au plus.

12. Outil à visser avec dispositif limitateur de couple transmis selon la revendication 1 à 11, **caractérisé en ce que** la présélection du couple de rotation pouvant être transmis tout au plus se fait de telle manière que par rotation du disque de réglage (6), la surface annulaire oblique (12) de ce dernier, en interaction avec la surface annulaire oblique (12) de la seconde boîte d'entraînement (5), provoque un déplacement axial de la seconde boîte d'entraînement (5) et de la seconde douille d'enclenchement (3b) et change ainsi la prétension du ressort de pression (9).

13. Outil à visser avec dispositif limitateur de couple transmis selon la revendication 1 à 12, **caractérisé en ce que** lors du réglage de la prétension du ressort (9) ou de la sélection du couple maximal, les dentures (6a) dans le disque de réglage (6) et dans le disque d'enclenchement (8), entraînant des déplacements axiaux de la seconde douille d'enclenchement (3b) et de la seconde boîte d'entraînement (5) contre la pression du ressort (9), glissent l'une sur l'autre par rotation du disque de réglage (6) et s'engrènent de nouveau par engagement positif dans la position choisie, laquelle est déterminée par la rotation et indiquée par la graduation.

14. Outil à visser avec dispositif limitateur de couple transmis selon la revendication 1 à 13, **caractérisé en ce que** la denture a un profil essentiellement triangulaire (21a), sur lequel l'angle de flanc α des dents - par rapport à l'axe de rotation - dans le sens de la rotatation lors du serrage des vis - vers la droite - est plus grand que dans le sens de la rotation lors du desserrage des vis - vers la gauche.

15. Outil à visser avec dispositif limitateur de couple transmis selon la revendication 1 à 14, **caractérisé en ce que** l'angle de flanc α des dents - par rapport à l'axe de rotation et dans le sens de rotation pour serrer les vis - est plus grand sur un tournevis destiné à transmettre un domaine de petits couples que sur un tournevis destiné à transmettre un domaine de grands couples.

16. Outil à visser avec dispositif limitateur de couple transmis selon la revendication 1 à 13, **caractérisé en ce que** la denture a un profil essentiellement triangulaire (21b), sur lequel l'angle de flanc α des dents dans le sens de rotation pour desserrer des vis - vers la gauche - est de 0° par rapport à l'axe de rotation.

17. Outil à visser avec dispositif limitateur de couple transmis selon la revendication 1 à 16, **caractérisé en ce que** les dentures (21) sont arrondies ou aplaties sur les sommets des dents.

18. Outil à visser avec dispositif limitateur de couple transmis selon la revendication 1 à 17, **caractérisé en ce que** les dentures (21) ont des dents avec un profil ondulé.

19. Outil à visser avec dispositif limitateur de couple transmis selon la revendication 1 à 18, **caractérisé en ce que** les dentures (2a, 5a, 15) sont des dentures rectangulaires.

20. Outil à visser avec dispositif limitateur de couple transmis selon la revendication 1 à 19, **caractérisé en ce que** le capuchon (20) et la poignée (14) sont vissés l'un sur l'autre.

21. Outil à visser avec dispositif limitateur de couple transmis selon la revendication 1 à 20, **caractérisé en ce que** la poignée (14) et le capuchon (20) sont assemblés de manière indissociable.

22. Outil à visser avec dispositif limitateur de couple transmis selon la revendication 1 à 21, **caractérisé en ce que** la douille dentée (2), les douilles d'enclenchement (3a, 3b), les boîtes d'entraînement (4, 5), le disque de réglage (6), le disque d'enclenchement (8), le ressort (9), la poignée (14) et le capuchon (20) sont disposés les uns par rapport aux autres et engrenés de telle manière que la force de prétension exercée par le ressort de pression (9) ne charge pas la douille dentée (2) en direction axiale.

23. Outil à visser avec dispositif limitateur de couple transmis selon la revendication 1 à 22, **caractérisé en ce que** des disques d'acier (13) sont disposés entre le ressort de pression (9) et les douilles d'enclenchement (3a, 3b).

24. Outil à visser avec dispositif limitateur de couple transmis selon la revendication 1 à 23, **caractérisé en ce que** tous les couples dans le domaine prédéterminé par un ressort (9) peuvent être réglés par rotation du disque de réglage (6), dans un domaine angulaire allant de 0° à 360°.

25. Outil à visser avec dispositif limitateur de couple transmis selon la revendication 1 à 24, **caractérisé en ce que** la force de prétension du ressort (9), charge le disque de réglage (6) et le disque d'enclenchement (8) à travers la seconde douille d'enclenchement (3b) et la seconde boîte d'entraînement (5).

26. Outil à visser avec dispositif limitateur de couple transmis selon la revendication 1 à 25, **caractérisé en ce que** la douille dentée (2), les douilles d'enclenchement (3a, 3b), les boîtes d'entraînement (4, 5), le disque de réglage (6), le disque d'enclenchement (8), la poignée (14) et le capuchon (20) sont fabriqués de préférence en matière plastique de type approprié, par le procédé de moulage par injection.

27. Outil à visser avec dispositif limitateur de couple transmis selon la revendication 1 à 26, **caractérisé en ce que** pour les pièces interagissant par glissement, respectivement une pièce est faite de polyamide et l'autre pièce de polyoxyméthylène, au moins une de ces matières plastiques étant compoundée avec du téflon.

28. Outil à visser avec dispositif limitateur de couple transmis selon la revendication 1 à 27, **caractérisé en ce que** les matières plastiques utilisées sont indéformables dans un domaine de températures allant jusqu'à environ 220 °C.

29. Outil à visser avec dispositif limitateur de couple transmis selon la revendication 1 à 27, **caractérisé en ce que** la douille dentée (2), les douilles d'enclenchement (3a, 3b), les boîtes d'entraînement (4, 5), le disque de réglage (6), le disque d'enclenchement (8), la poignée (14), le capuchon (20) ou certaines de ces pièces sont en métal.

30. Outil à visser avec dispositif limitateur de couple transmis selon la revendication 1 à 25 et 29, **caractérisé en ce qu'**en coupe transversale, le contour extérieur de la poignée (14) en métal est ovalisé, et **en ce qu'**une gaine de poignée (22) en matière plastique et de forme ergonomique est enfilée sur la poignée (14), de manière à pouvoir être remplacée.

31. Outil à visser avec dispositif limitateur de couple transmis selon la revendication 1 à 30, **caractérisé en ce que** la tige (1) placée dans la douille dentée (2) est inamovible.

32. Outil à visser avec dispositif limitateur de couple transmis selon la revendication 1 à 30, **caractérisé en ce que** la tige (1) est placée dans la douille dentée (2) d'une manière échangeable, et **en ce qu'**elle est tenue axialement par un dispositif de serrage (18, 19) qui a été façonné sur la douille dentée en ne formant qu'un seul bloc avec elle.

33. Outil à visser avec dispositif limitateur de couple transmis selon la revendication 1 et 32, **caractérisé en ce que** la tige (1) est reliée d'une manière non rotative à la douille dentée (2), grâce à un profil ovalisé en coupe transversale ou grâce à des élements d'entraînement conçus autrement, **en ce que** la tige (1) s'étend jusqu'à proximité de l'extrémité arrière de la douille dentée, et **en ce qu'**elle y trouve un appui axial.
